# EUROPEAN PATENT APPLICATION

(11) **EP 4 501 974 A1**
(43) Date of publication of application: **05.02.2025**
(21) Application number: 23775111.0
(22) Date of filing: 24.03.2023
(51) Int. Cl.: C08F 4/40, A61K 6/60, C08F 2/44

(54) **POLYMERIZATION ACCELERATOR, POLYMERIZATION INITIATOR, KIT FOR PREPARING CURABLE COMPOSITION, CURABLE COMPOSITION, CURED PRODUCT AND DENTAL MATERIAL**

(30) Priority: 25.03.2022 JP 2022050803
(71) Applicant: Mitsui Chemicals, Inc., Tokyo 104-0028 (JP)
(72) Inventor: TAKAHASHI, Issei, Sodegaura-shi, Chiba 299-0265 (JP); DANJO, Takahiro, Sodegaura-shi, Chiba 299-0265 (JP)
(74) Representative: Hoffmann Eitle
(86) International application number: PCT/JP2023/012011
(87) International publication number: WO 2023/182518

(57) **Abstract**

A polymerization accelerator contains a phosphonite compound.

## Description

### Technical Field

The present disclosure relates to a polymerization accelerator, a polymerization initiator, a curable composition preparation kit, a curable composition, a cured product, and a dental material. Background technology

In the dental field, a synthetic resin molding product or the like is used to repair a tooth defect. For example, a curable composition called cement is used as a tooth substitute for repairing a large tooth defect. In recent years, the scope of use of cement has expanded.

A photopolymerization initiator, a chemical polymerization initiator, or the like may be used for polymerization of a curable composition for dental materials such as cement.
For example, one of the common chemical polymerization initiator types is a redox type polymerization initiator that combines an oxidizing agent and a reducing agent. As a redox type polymerization initiator, for example, a polymerization initiator type using an organic peroxide as an oxidizing agent and an aromatic amine compound as a reducing agent is known.

Patent Document 1 discloses a redox initiator type containing sulfite.
[Patent Document 1] International Publication No. 2017/100231

### SUMMARY OF INVENTION

### Technical Problem

However, the polymerization initiator described in Patent Document 1 has room for improvement in terms of monomer polymerizability.

The problem to be solved by a one embodiment of the present disclosure is to provide a polymerization accelerator, a polymerization initiator, a curable composition preparation kit, a curable composition, a cured product, and a dental material that can suitably improve the monomer polymerizability.

### Solution to Problem

The specific means to solve the above problem include the following aspects.
<1> A polymerization accelerator, comprising a phosphonite compound.
<2> The polymerization accelerator according to <1>, wherein the phosphonite compound includes a structure represented by the following general formula (X). 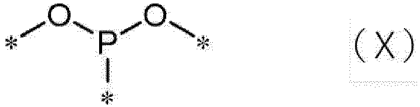 In the general formula (X), * is a bonding position with a carbon atom.
<3> The polymerization accelerator according to <1> or <2>, wherein the phosphonite compound contains a compound represented by the following general formula (Y). 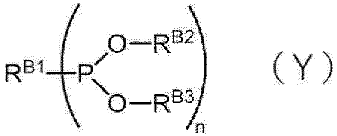 In the general formula (Y), R^{B1} represents an n-valent hydrocarbon group, each of R^{B2} and R^{B3} independently represents a monovalent hydrocarbon group, and n is an integer 1 or 2.
<4> A polymerization initiator, comprising the phosphonite compound according to any one of <1> to <3>, an oxidizing agent and a reducing agent.
<5> A curable composition preparation kit, comprising:
   a first agent containing a monomer (1), and
   a second agent containing a monomer (2),
   wherein each of the phosphonite compound according to any one of <1> to <3>, an oxidizing agent and a reducing agent are independently contained in at least one of the first agent or the second agent.
<6> The curable composition preparation kit according to <5>, wherein:
   the oxidizing agent contains an organic peroxide, and
   the reducing agent contains an ascorbic acid-type compound and a transition metal compound.
<7> The curable composition preparation kit according to <6>, wherein:
   the first agent contains the transition metal compound and the organic peroxide, and
   the second agent contains the ascorbic acid-type compound and the phosphonite compound.
<8> The curable composition preparation kit according to any one of <5> to <7>, wherein one of the monomer (1) contained in the first agent or the monomer (2) contained in the second agent contains an acidic group-containing monomer.
<9> The curable composition preparation kit according to <6> or <7>, wherein:
   the monomer (1) contained in the first agent contains an acidic group-containing monomer, and
   the second agent contains the ascorbic acid-type compound and the phosphonite compound.
<10> The curable composition preparation kit according to any one of <6> to <9>, wherein the second agent contains the ascorbic acid-type compound and the phosphonite compound.
<11> The curable composition preparation kit according to any one of <5> to <10>, wherein the first agent and the second agent contain a filler.
<12> The curable composition preparation kit according to any one of <5> to <11>, wherein a total content of the phosphonite compound contained in the first agent and the second agent is from 0.1% by mass to 1.5% by mass with respect to a total amount of a curable composition to be prepared.
<13> A curable composition, comprising the phosphonite compound according to any one of <1> to <3>, an oxidizing agent, a reducing agent and a monomer.
<14> A cured product of the curable composition according to <13>.
<15> A dental material, comprising the cured product according to <14>.

### Advantageous Effects of Invention

According to one embodiment of the present disclosure, a polymerization accelerator, a polymerization initiator, a curable composition preparation kit, a curable composition, a cured product, and a dental material that can suitably improve the monomer polymerizability can be provided.

### DESCRIPTION OF EMBODIMENTS

In the present disclosure, those numerical ranges that are expressed with "to" each denote a range that includes the numerical values stated before and after "to" as the minimum value and the maximum value, respectively.

In a set of numerical ranges that are stated stepwise in the present disclosure, the upper limit value or the lower limit value of a numerical range may be replaced with the upper limit value or the lower limit value of other numerical range. Further, in a numerical range stated in the present disclosure the upper limit value or the lower limit value of the numerical range may be replaced with a value indicated in Examples.

In the present disclosure, a combination of two or more preferred embodiments is a more preferred embodiment.

In the present disclosure, when there are plural kinds of substances that correspond to a component, the indicated content of the component means, unless otherwise specified, the total content of the plural kinds of substances.

In the present disclosure, "(meth)acrylic" means acrylic and methacrylic, and "(meth)acryloyl" means acryloyl and methacryloyl.

In the present disclosure, "A or B" means at least one of A or B, and may include both A and B (for example, a mixture of A and B).

### <<Polymerization accelerator>>

A polymerization accelerator of the present disclosure contains a phosphonite compound. By using a polymerization accelerator containing a phosphonite compound, the polymerizability of a monomer can be favorably improved. The polymerization accelerator of the present disclosure may contain one or more types of phosphonite compounds, or may contain two or more types of phosphonite compounds.

### <Phosphonite compound>

The phosphonite compound may be a trivalent organic phosphorus compound in which a carbon atom is bonded to a phosphorus atom. The phosphonite compound contained in the polymerization accelerator of the present disclosure preferably includes a structure represented by the following general formula (X).

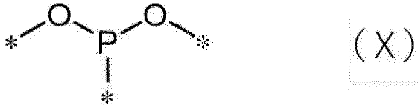

In the general formula (X), * is a bonding position with a carbon atom. Each of the three * is preferably a bonding position with a carbon atom included in a hydrocarbon group, and more preferably a bonding position with a carbon atom included in a benzene ring.

The phosphonite compound may include one structure represented by the general formula (X), or may include two or more structures represented by the general formula (X). The phosphonite compound preferably includes two structures represented by the general formula (X), and more preferably includes a skeleton in which two structures represented by the general formula (X) are bonded via a divalent linking group (preferably a biphenyl structure).

The phosphonite compound contained in the polymerization accelerator of the present disclosure preferably contains a compound represented by the following general formula (Y).

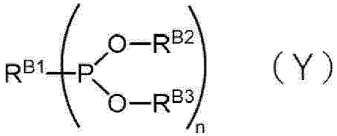

In the general formula (Y), R^{B1} represents an n-valent hydrocarbon group, each of R^{B2} and R^{B3} independently represents a monovalent hydrocarbon group, and n is an integer 1 or 2.

Examples of the n-valent hydrocarbon group in R^{B1} include an n-valent aliphatic hydrocarbon group, an n-valent alicyclic hydrocarbon group, an n-valent aromatic hydrocarbon group, a combination of two or more of these, or the like.

A monovalent hydrocarbon group in R^{B1} is preferably an alkyl group, a phenyl group, a biphenyl group, or the like. A hydrogen atom of the phenyl group or biphenyl group in R^{B1} may be substituted with a substituent such as an alkyl group.

A divalent hydrocarbon group in R^{B1} is preferably a biphenylene group such as an alkylene group, a phenylene group, a 4,4'-biphenylene group, a 4,3'-biphenylene group, or a 3,3'-biphenylene group. A hydrogen atom included in the phenylene group or biphenylene group in R^{B1} may be substituted with a substituent such as an alkyl group.

Each of R^{B2} and R^{B3} independently represents a monovalent hydrocarbon group, and the monovalent hydrocarbon group is preferably an alkyl group, a phenyl group or the like. A hydrogen included in the phenyl group in R^{B2} and R^{B3} may be substituted with an alkyl group such as a tert-butyl group or n-butyl group.

n is an integer of 1 or 2, preferably 2.

Specific examples of the phosphonite compound contained in the polymerization accelerator of the present disclosure include, for example, tetrakis(2,4-di-tert-butylphenyl)4,4'-biphenylene-di-phosphonite, tetrakis(2,4-di-tert-butyl-5-methylphenyl)4,4'-biphenylene-di-phosphonite, tetrakis(2,4-di-tert-butylphenyl)4,3'-biphenylene-di-phosphonite, tetrakis(2,4-di-tert-butylphenyl)3,3'-biphenylene-di-phosphonite, tetrakis(2,6-di-tert-butylphenyl)-4,4'-biphenylene-di-phosphonite, tetrakis(2,6-di-tert-butylphenyl)-4,3'-biphenylene-di-phosphonite, tetrakis(2,6-di-tert-butylphenyl)-3,3'-biphenylene-di-phosphonite, bis(2,4- di-tert-butylphenyl)-4-phenyl-phenylphosphonite, bis(2,4-di-tert-butylphenyl)-3-phenyl-phenylphosphonite, bis(2,6-di-n-butylphenyl)-3-phenyl-phenylphosphonite, bis(2,6-di-tert-butylphenyl)-4-phenyl-phenylphosphonite, bis(2,6-di-tert-butylphenyl)-3-phenyl-phenylphosphonite or the like.

The polymerization accelerator of the present disclosure may be a polymerization accelerator consisting only of a phosphonite compound, or may be a polymerization accelerator consisting of a phosphonite compound and another polymerization accelerator. Examples of another polymerization accelerator include a phosphite compound, a sulfite compound, an inorganic salt, thiourea, or the like.

When using another polymerization accelerator, one type may be used alone, or two or more types may be used in combination.

Examples of the phosphite compound include triphenyl phosphite, trisnonylphenyl phosphite, tricresyl phosphite, diphenyl mono(2-ethylhexyl) phosphite, diphenyl monodecyl phosphite, diphenyl mono(tridecyl) phosphite, tris(2,4-di-tert-butylphenyl) phosphite, or the like.

Examples of the sulfite compound include ethylene sulfite, propylene sulfite, dimethyl sulfite, diethyl sulfite, ethyl methyl sulfite, methyl-n-propyl sulfite, ethyl-n-propyl sulfite, di-n-propyl sulfite, diphenyl sulfite, methylphenyl sulfite, ethyl sulfite, dibenzyl sulfite, benzylmethyl sulfite, benzylethyl sulfite, or the like.

Examples of the inorganic salt include sodium sulfite, calcium sulfite, potassium sulfite, potassium nitrate, potassium chloride, potassium sulfate, sodium chloride or the like.

Examples of thiourea include acetylthiourea, phenylthiourea, triethylthiourea, tetramethylthiourea, dimethylthiourea, diphenylthiourea, or the like.

When the polymerization accelerator of the present disclosure consists of a phosphonite compound and another polymerization accelerator, the content of the phosphonite compound may be from 50% by mass to 99% by mass, may be from 60% by mass to 98% by mass, or may be from 70% by mass to 97% by mass, with respect to the total amount of the polymerization accelerator.

When the polymerization accelerator of the present disclosure consists of a phosphonite compound and another polymerization accelerator, the content of another polymerization accelerator may be from 0.5% by mass to 30% by mass, may be from 1% by mass to 20% by mass, or may be from 1.5% by mass to 10% by mass, with respect to the total amount of the polymerization accelerator.

### <<Polymerization initiator>>

A polymerization initiator of the present disclosure contains the aforementioned phosphonite compound or the polymerization accelerator of the present disclosure, an oxidizing agent, and a reducing agent. The polymerization initiator of the present disclosure is a redox polymerization initiator that combines an oxidizing agent and a reducing agent.

Preferred embodiments of the phosphonite compound or polymerization accelerator contained in the polymerization initiator of the present disclosure are as described above. In the polymerization initiator of the present disclosure, the content of the phosphonite compound or the content of the polymerization accelerator is preferably from 1 part by mass to 40 parts by mass, more preferably from 3 parts by mass to 30 parts by mass, and still more preferably from 5 parts by mass to 20 parts by mass, with respect to 100 parts by mass of the polymerization initiator.

### <Oxidizing agent>

The polymerization initiator of the present disclosure includes an oxidizing agent. The oxidizing agent is not particularly limited as long as it can be used as a redox polymerization initiator, and examples thereof include a peroxide.

Examples of the peroxide include an organic peroxide and an inorganic peroxide, and the peroxide in the present disclosure preferably includes an organic peroxide.

### (Organic peroxide)

Known organic peroxides can be used without a particular limitation. Examples of typical organic peroxides include hydroperoxide, peroxyester, ketone peroxide, peroxyketal, dialkyl peroxide, diacyl peroxide, peroxydicarbonate, or the like. Among these, hydroperoxide is preferable because even if the curable composition is provided in a packaged form and stored for a long period of time, there is little variation in the operable time. One type of organic peroxide may be used alone, or a plurality of types may be used in combination.

More specifically, examples of the hydroperoxide include cumene hydroperoxide, t-butyl hydroperoxide, t-hexyl hydroperoxide, p-menthane hydroperoxide, diisopropylbenzene hydroperoxide, 1,1,3,3-tetramethylbutyl hydroperoxide and t-amyl hydroperoxide.

As the peroxy ester, any known peroxy ester may be used without any restriction as long as it includes an acyl group on one side of a peroxy group (-O-O- group) and a hydrocarbon group (or a similar group) on the other side. The specific examples include α,α-bis(neodecanoylperoxy)diisopropylbenzene, cumylperoxyneodecanoate, 1,1,3,3-tetramethylbutylperoxyneodecanoate, 1-cyclohexyl-1-methyl ethyl peroxy neodecanoate, t-hexyl peroxy neodecanoate, t-butyl peroxy neodecanoate, t-hexyl peroxy pivalate, t-butyl peroxy pivalate, 1,1,3,3-tetramethylbutylperoxy-2-ethylhexanoate, 2,5-dimethyl-2,5-bis(2-ethylhexanoylperoxy)hexane, 1-cyclohexyl-1-methylethylperoxy-2-ethylhexanoate, t-hexylperoxy 2-ethylhexanoate, t-butylperoxy 2-ethylhexanoate, t-butylperoxyisobutyrate, t-hexylperoxyisopropyl monocarbonate, t-butylperoxymaleic acid, t-butylperoxy 3,5,5-trimethylhexanoate, t-butylperoxylaurate, 2,5-dimethyl-2,5-bis(m-toluoylperoxy)hexane, t-butylperoxyisopropyl monocarbonate, t-butylperoxy 2-ethylhexyl monocarbonate, t-hexylperoxybenzoate, 2,5-dimethyl-2,5-bis(benzoylperoxy)hexane, t -butylperoxyacetate, t-butylperoxy-m-toluoylbenzoate, t-butylperoxybenzoate, bis(t-butylperoxy)isophthalate or the like. These can be used alone or in a combination of two or more as appropriate.

Examples of the ketone peroxide include methyl ethyl ketone peroxide, cyclohexanoperoxide, methylcyclohexanone peroxide, methyl acetoacetate peroxide, and acetylacetone peroxide or the like.

Examples of the peroxyketal include 1,1-bis(t-hexylperoxy)3,3,5-trimethylcyclohexane, 1,1-bis(t-hexylperoxy)cyclohexane, 1,1-bis(t-butyl peroxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, 2,2-bis(4,4-di-t-butylperoxycyclohexyl)propane, or the like.

Examples of the dialkyl peroxide include α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexane-3, or the like.

Examples of the diacyl peroxide include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, or a benzoyl peroxide-type compound.

Examples of the peroxydicarbonate include di-n-propyl peroxydicarbonate, diisopropyl peroxydicarbonate, bis(4-t-butylcyclohexyl) peroxydicarbonate, di-2-ethoxyethyl peroxydicarbonate, di-2-ethylhexyl peroxydicarbonate, di-2-methoxybutyl peroxydicarbonate, di(3-methyl-3-methoxybutyl)peroxydicarbonate, or the like.

### (Inorganic Peroxide)

Examples of the inorganic peroxide include peroxodisulfate, peroxodiphosphate or the like, and among these, peroxodisulfate is preferable from the viewpoint of curability. Specific examples of the peroxodisulfate include sodium peroxodisulfate, potassium peroxodisulfate, aluminum peroxodisulfate, and ammonium peroxodisulfate.

The above peroxodisulfate may be used alone or in combination of multiple types. Among the above peroxodisulfate, sodium peroxodisulfate, potassium peroxodisulfate, and ammonium peroxodisulfate are preferable.

In the polymerization initiator of the present disclosure, the content of the oxidizing agent is preferably from 20 parts by mass to 90 parts by mass, more preferably from 30 parts by mass to 80 parts by mass, and still more preferably from 40 parts by mass to 70 parts by mass, with respect to 100 parts by mass of the polymerization initiator.

### <Reducing agent>

The polymerization initiator of the present disclosure contains a reducing agent. The reducing agent is not particularly limited as long as it can be used in a redox polymerization initiator, and examples thereof include ascorbic acid-type compound, a sulfinic acid-type compound, an amine compound, a transition metal compound, or the like.

### (Ascorbic acid-type compound)

Examples of the ascorbic acid-type compound that is a reducing agent include, for example, ascorbic acid, an ascorbic acid derivative, salts thereof, or the like. From the viewpoint of improving the adhesiveness of the resulting cured product, it is preferable to use an ascorbic acid derivative or a salt thereof.

Examples of the salt of ascorbic acid include an alkali metal salt of ascorbic acid, an alkaline earth metal salt of ascorbic acid, or the like, and more specifically, example thereof include a sodium salt, a potassium salt, a magnesium salt, and a calcium salt of ascorbic acid or the like.

Examples of the ascorbic acid derivative include a compound in which a part of the structure of the ascorbic acid skeleton is substituted, and a compound in which introduction of substituent, oxidation, reduction, atomic substitution or the like is performed to ascorbic acid. The ascorbic acid derivative may be a compound in which at least one of the four hydroxyl groups included in ascorbic acid is substituted with another group.

The ascorbic acid derivative preferably includes a structure in which at least one of the hydroxyl groups included in ascorbic acid is substituted with an ether bond bonded to a carbon atom.

Examples of the salt of ascorbic acid derivative include an alkali metal salt of ascorbic acid derivative, an alkaline earth metal salt of ascorbic acid derivative or the like, and more specifically, example thereof include a sodium salt, a potassium salt, a magnesium salt, and a calcium salt of ascorbic acid derivative, or the like. Among these, the calcium salt is preferable from the viewpoint of balance between polymerizability and adhesiveness.

The ascorbic acid derivative or salt thereof may include a structure in which at least one of the four hydroxyl groups included in ascorbic acid is substituted with an ether bond bonded to a carbon atom. It is preferable that it includes a structure in which each of two of the four hydroxyl groups included in ascorbic acid is substituted with an ether bond bonded to a carbon atom.

The ascorbic acid derivative preferably includes a structure represented by the following general formula (A).

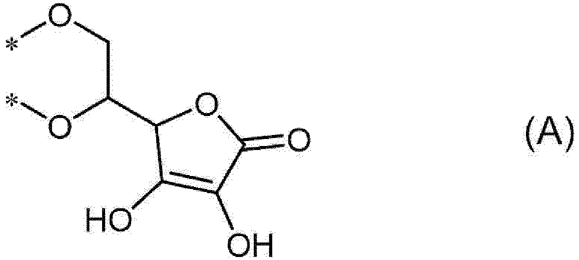

In the general formula (A), * represents the bonding position with a carbon atom. Two *'s in the general formula (A) may be bonded to the same carbon atom to form a 1,3-dioxolane skeleton.

The ascorbic acid derivative preferably includes a compound represented by the following general formula (B).

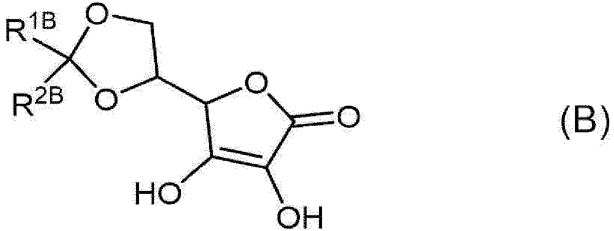

In the general formula (B), R^{1B}and R^{2B} are each independently a hydrogen atom or a monovalent organic group.

At least one of R^{1B} or R^{2B} is preferably a monovalent organic group.

The monovalent organic group in R^{1B} and R^{2B} is preferably an organic group having 1 to 12 carbon atoms, more preferably an organic group having 1 to 10 carbon atoms, and still more preferably an organic group having 3 to 10 carbon atoms.

In the general formula (B), one of R^{1B}or R^{2B} may be a hydrogen atom, and the other of R^{1B} or R^{2B} may be a monovalent organic group having 1 to 10 carbon atoms.

The monovalent organic group in R^{1B} and R^{2B} may be an organic group including an oxygen atom, a nitrogen atom, a sulfur atom, or the like, may be a hydrocarbon group to which an oxygen atom, a nitrogen atom, a sulfur atom, or the like is bonded, or may be a hydrocarbon group.

The salt of the ascorbic acid derivative may be a salt of a compound represented by the general formula (B), or may be an alkali metal salt or an alkaline earth metal salt of a compound represented by the general formula (B). From the viewpoint of balance between polymerizability and adhesive properties, a calcium salt of a compound represented by the general formula (B) is preferable.

When the salt of an ascorbic acid derivative is a salt of a compound represented by the general formula (B), each of R^{1B} and R^{2B} is independently preferably a hydrogen atom or a monovalent organic group. Furthermore, it is preferable that one of R^{1B} or R^{2B} is a hydrogen atom, and the other of R^{1B} or R^{2B} is a monovalent organic group having 1 to 10 carbon atoms, or each of R^{1B} and R^{2B} is independently a monovalent organic group having 1 to 10 carbon atoms.

When one of R^{1B} or R^{2B} is a hydrogen atom, and the other of R^{1B} or R^{2B} is a monovalent organic group having 1 to 10 carbon atoms, the other of R^{1B} or R^{2B} is preferably a monovalent organic group having 1 to 5 carbon atoms, more preferably a monovalent organic group having 1 to 3 carbon atoms, still more preferably a methyl group, an ethyl group, a propyl group or isopropyl group and particularly preferably an isopropyl group.

When each of R^{1B} and R^{2B} is independently a monovalent organic group having 1 to 10 carbon atoms, each of R^{1B} and R^{2B} is independently preferably a monovalent organic group having 1 to 5 carbon atoms, more preferably a monovalent organic group having 1 to 3 carbon atoms, still more preferably a methyl group, an ethyl group, a propyl group, or an isopropyl group and it is particularly preferable that both of them are methyl groups.

Examples of a method of producing an ascorbic acid derivative, include a method of reacting ascorbic acid represented by the following general formula (C) with an aldehyde compound represented by the following general formula (D) or a ketone compound represented by the following general formula (E) by acetalization (see dashed line).

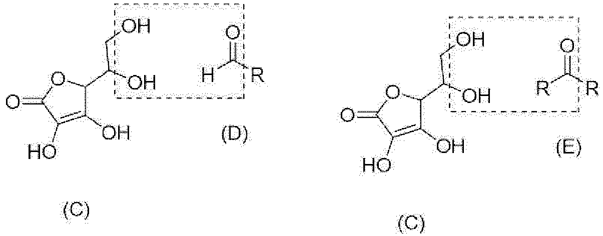

R in the above general formula (D) is the same as R^{1B}or R^{2B} in the aforementioned general formula (B).

Two R's in the above general formula (E) are the same as R^{1B}or R^{2B} in the aforementioned general formula (B). The two R's in the above general formula (E) may be the same or different.

Examples of the aldehyde compound represented by the above general formula (D) include butanal, isobutanal, hexanal, octanal, dodecanal, or the like. Examples of the ketone compound represented by the above general formula (E) include acetone, methyl ethyl ketone, methyl isobutyl ketone or the like.

These compounds may be used alone or in combination of two or more.

Examples of a method of producing an alkali metal salt of an ascorbic acid derivative, which is a salt of the ascorbic acid derivative, include a method (alkali metalation reaction) of reacting an ascorbic acid derivative represented by the following general formula (B) with an alkali metal salt represented by the following general formula (F).

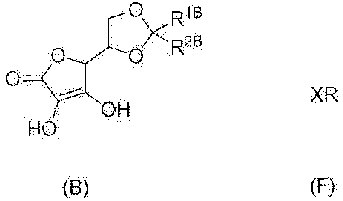

In the general formula (F), X represents an alkali metal, and R represents, for example, a carbonate group or a hydroxyl group.

Examples of the alkali metal salt represented by the above general formula (F) include sodium hydroxide, sodium hydrogen carbonate, sodium carbonate, calcium hydroxide, calcium carbonate or the like. These may be used alone or in combination of two or more.

Regarding the acetalization reaction and alkali metalization reaction described above, the reaction conditions may be adjusted as appropriate.

### (Sulfinic acid-type compound)

Examples of the sulfinic acid-type compound that is a reducing agent include a sulfinic acid compound, a salt thereof or the like.

Examples of the sulfinic acid compound or the salt thereof include an alkanesulfinic acid or a salt thereof, an alicyclic sulfinic acid or a salt thereof, and an aromatic sulfinic acid or a salt thereof.

Examples of the salt of a sulfinic acid compound include a lithium salt, a sodium salt, a potassium salt, a rubidium salt, a cesium salt, a magnesium salt, a calcium salt, a strontium salt, an iron salt, a zinc salt, an ammonium salt, a tetramethylammonium salt, or a tetraethylammonium salt of the sulfinic acid compound, or the like.

Examples of the alkanesulfinic acid include methanesulfinic acid or the like.

Examples of the alicyclic sulfinic acid include cyclohexane sulfinic acid, cyclooctane sulfinic acid or the like.

Examples of the aromatic sulfinic acids include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid or the like.

### (Amine compound)

Examples of the amine compound that is a reducing agent include an aromatic amine compound, an aliphatic amine compound, a heterocyclic amine compound or the like.

From the viewpoint of improving polymerizability, the amine compound preferably contains an aromatic amine compound.

The aromatic amine compound includes an aromatic hydrocarbon group in its structure. Further, a heterocyclic aromatic compound is not classified as the aromatic amine compound in the present disclosure.

Examples of the aromatic amine compound include an aromatic primary amine compound such as aniline and toluidine;
an aromatic secondary amine compound such as an aromatic substituted amino acid compound or a salt thereof which is as represented by an aromatic substituted glycine such as N-methylaniline, N-methyl-p-toluidine, N-phenylglycine (NPG), N-tolylglycine (NTG), and N,N-(3-methacryloyloxy-2-hydroxypropyl)phenylglycine (NPG-GMA) or its alkali metal salt, alkaline earth metal salt, amine salt, ammonium salt, or the like;
an aromatic tertiary amine compound or a salts thereof such as N,N-dimethylaniline (DMA), N,N-dibenzylaniline, N,N-dimethyl-p-toluidine (DMPT), N,N-diethyl-p-toluidine, N,N-di(2-hydroxy ethyl)-p-toluidine (DEPT), N,N-dimethyl-p-ethylaniline, N,N-dimethyl-p-isopropylaniline, N,N-dimethyl-p-tert-butylaniline, N,N-dimethyl anisidine, N,N-dimethylxylidine, N,N-dimethyl-3,5-dit-butylaniline, N,N-dimethyl-p-chloroaniline, N,N-dimethyl-p-fluoroaniline, or the like.

As the aliphatic amine compound, an aliphatic amine compound including at least one selected from the group consisting of primary, secondary and tertiary amino groups is preferable, and an aliphatic amine compound including only a tertiary amino group is more preferable.

As the heterocyclic amine compound, a heterocyclic amine compound including at least one selected from the group consisting of primary, secondary, and tertiary amino groups is preferable, and a heterocyclic amine compound including only a tertiary amino group is more preferable.

Examples of the aliphatic amine compound include EDTA type (ethylenediaminetetraacetic acid), NTA type (nitrilotetraacetic acid), DTPA type (diethylenetriaminepentaacetic acid), HEDTA type (hydroxyethylethylenediaminetriacetic acid), and TTHA type (triethylenetetraminehexaacetic acid), PDTA type (1,3-propanediaminetetraacetic acid), DPTA-OH type (1,3-diamino-2-hydroxypropane tetraacetic acid), HIDA type (hydroxyethyliminodiacetic acid), DHEG type (dihydroxyethyl glycine), GEDTA type (glycol ether diamine tetraacetic acid), CMGA type (dialboxymethylglutamic acid), EDDS type ((S,S)-ethylenediamine disuccinic acid) and EDTMP type (ethylenediaminetetra(methylenephosphonic acid)), N,N-dimethyl-N',N'-bis(2-dimethylaminoethyl)ethylenediamine (Me6TREN), N,N'-dimethyl-1,2-phenylenediamine, 2-(methylamino)phenol, 3-( methylamino)-2-butanol, N,N'-bis(1,1-dimethylethyl)-1 ,2-ethanediamine or N,N,N',N",N"-pentamethyldiethylenetriamine (PMDETA) or salts thereof or the like.

Examples of the heterocyclic amine compound include a heterocyclic amine compound that can function as a monodentate, bidentate, or tridentate heterocyclic electron donor ligand.

Specifically, examples thereof include a heterocyclic amine compound derived from an unsubstituted or substituted heteroarene such as furan, thiophene, pyrrole, pyridine, bipyridine, picolilimine, γ-pyran, γ-thiopyran, phenanthroline, pyrimidine, bis-pyrimidine, pyrazine, indole, coumarin, thionaphthene, carbazole, dibenzofuran, dibenzothiophene, pyrazole, imidazole, benzimidazole, oxazole, thiazole, bis-thiazole, isoxazole, isothiazole, quinoline, biquinoline, isoquinoline, biisoquinoline, acridine, chroman, phenazine, phenoxazine, phenothiazine, triazine, thianthrene, purine, bismidazole, bisoxazoline, or the like or a salt thereof.

The aliphatic amine compound or heterocyclic amine compound is preferably N,N-dimethyl-N',N'-bis(2-dimethylaminoethyl)ethylenediamine, 2,2'-bipyridine, N-butyl-2-pyridylmethanimine, 4,4'-di-tert-butyl-2,2'-dipyridine, 4,4'-dimethyl-2,2'-dipyridine, 4,4'-dinonyl-2,2'-dipyridine, N-dodecyl-N-(2-pyridylmethylene)amine, 1,1,4,7,10,10-hexamethyl-triethylenetetramine, N-octadecyl-N-(2-pyridylmethylene)amine, N-octyl-2-pyridylmethanimine, N,N,N',N",N"-pentamethyl-diethylenetriamine, 1,4,8,11-tetracyclotetradecane, N,N,N',N'-tetrakis(2-pyridyl methyl)ethylenediamine, 1,4,8,11-tetramethyl-1,4,8,11-tetraazacyclotetradecane, tris[2-(diethylamino)ethyl]amine or tris(2-methylpyridyl)amine, and more preferably N,N-dimethyl-N',N'-bis(2-dimethylaminoethyl)ethylenediamine (Me6TREN).

### (Transition metal compound)

Examples of the transition metal compound that is a reducing agent include a copper compound, a vanadium compound, a molybdenum compound, a scandium compound, a titanium compound, a chromium compound, a manganese compound, an iron compound, a cobalt compound, a nickel compound, or the like.

Among the above, the transition metal compound preferably contains at least one of a copper compound or a vanadium compound, and more preferably contains a copper compound.

In the copper compound, examples of a copper carboxylate include copper acetate, copper isobutyrate, copper gluconate, copper citrate, copper phthalate, copper tartrate, copper oleate, copper octylate, copper octenoate, copper naphthenate, copper methacrylate, copper 4-cyclohexylbutyrate; examples of a β-diketone copper include, copper acetylacetone, copper trifluoroacetylacetone, copper hexafluoroacetylacetone, copper 2,2,6,6-tetramethyl-3,5-heptanedionato, copper benzoylacetone; examples of a β-ketoester copper include ethyl copper acetoacetate; examples of copper alkoxide include copper methoxide, copper ethoxide, copper isopropoxide, copper 2-(2-butoxyethoxy)ethoxide, copper 2-(2-methoxyethoxy)ethoxide; examples of a copper dithiocarbamate include copper dimethyldithiocarbamate; examples of the salt of copper and an inorganic acid include copper nitrate; and copper chloride.

Among these, from the viewpoint of solubility and reactivity with a monomer, a copper carboxylate, a β-diketone copper, and a β-ketoester copper are preferable, and copper acetate and copper acetylacetone are more preferable.

Examples of the vanadium compound include vanadyl acetylacetonate, vanadium (III) naphthenate, vanadyl stearate, vanadium benzoylacetonate, bis(maltolate)oxovanadium (IV), oxobis(1-phenyl-1,3-butanedionate)vanadium (IV) or the like.

In the polymerization initiator of the present disclosure, the content of the reducing agent is preferably from 10 parts by mass to 60 parts by mass, more preferably from 15 parts by mass to 55 parts by mass, and still more preferably from 20 parts by mass to 50 parts by mass, with respect to 100 parts by mass of the polymerization initiator.

When the polymerization initiator of the present disclosure contains an ascorbic acid-type compound as a reducing agent, the content of the ascorbic acid-type compound in the polymerization initiator of the present disclosure is preferably from 10 parts by mass to 60 parts by mass, more preferably from 15 parts by mass to 55 parts by mass, and still more preferably from 20 parts by mass to 50 parts by mass, with respect to 100 parts by mass of the polymerization initiator.

When the polymerization initiator of the present disclosure contains a transition metal compound as a reducing agent, the content of the transition metal compound in the polymerization initiator of the present disclosure is preferably from 0.001 parts by mass to 1 part by mass, more preferably from 0.005 parts by mass to 0.8 parts by mass, and still more preferably from 0.01 parts by mass to 0.7 parts by mass, with respect to 100 parts by mass of the polymerization initiator.

### «Curable composition preparation kit»

A curable composition preparation kit of the present disclosure includes a first agent containing a monomer (A) and a second agent containing a monomer (B), and each of the aforementioned phosphonite compound or the polymerization accelerator of the present disclosure, an oxidizing agent and a reducing agent are independently contained in at least one of the first agent or the second agent.

### <Monomer>

In the curable composition preparation kit of the present disclosure, the first agent contains a monomer (A), and the second agent contains a monomer (B).

The monomer (A) and monomer (B) may be the same monomer or may be different monomers.

As the monomer (A) and monomer (B), known monomers can be used.

The monomer (A) and monomer (B) may be a monomer that does not include an acidic group or may be a monomer that includes an acidic group (hereinafter also referred to as "acidic group-containing monomer").

It is preferable that the monomer (A) and monomer (B) contains a monomer that does not include an acidic group.

The monomer is a monomer that undergoes a radical polymerization reaction and becomes a polymer under the action of the aforementioned phosphonite compound or the polymerization accelerator of the present disclosure, an oxidizing agent, and a reducing agent.

The monomer constituting the monomer in the present disclosure is not limited to one type, and two or more types may be used. Examples of the monomer that does not include an acidic group include a (meth)acrylate monomer that does not include an acidic group. Examples of the (meth)acrylate monomer that does not include an acidic group include a monofunctional monomer, a bifunctional monomer, or a trifunctional or higher functional monomer.

In the present disclosure, the content of the monomer (i.e., the total amount of the monomer (A) and monomer (B) in the curable composition to be prepared) is preferably from 10% by mass to 90% by mass, more preferably from 20% to 75% by mass, and still more preferably from 30% to 60% by mass, with respect to the total amount of the curable composition to be prepared.

Examples of the monofunctional monomer include 2-hydroxyethyl (meth)acrylate, 3-hydroxypropyl (meth)acrylate, 2-hydroxypropyl (meth)acrylate, 1,3-dihydroxypropyl (meth)acrylate, and 2,3-dihydroxypropyl (meth)acrylate. Among these, 2-hydroxyethyl methacrylate (HEMA) is preferable.

Examples of an aromatic compound-typed difunctional monomer include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxypolypropoxyphenyl)propane, or the like.

Among these, 2,2-bis[4-(3-(methacryloyloxy)-2-hydroxypropoxyphenyl)propane (commonly known as "Bis-GMA"), and 2,2-bis(4-methacryloyloxypolyethoxyphenyl)propane are preferable.

Examples of an aliphatic compound-typed difunctional monomer include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate , 1,10-decanediol di(meth)acrylate, 2,2,4-trimethylhexamethylenebis(2-carbamoyloxyethyl)dimethacrylate (UDMA), 1,2-bis(3-methacryloyloxy-2-hydroxypropyloxy)ethane or the like.

Among these, glycerol dimethacrylate, triethylene glycol dimethacrylate (TEGDMA), 1,6-hexanediol dimethacrylate (HexDMA), neopentyl glycol dimethacrylate (NPG), 2,2,4-trimethylhexamethylene bis(2-carbamoyloxyethyl)dimethacrylate (UDMA) and 1,2-bis(3-methacryloyloxy-2-hydroxypropyloxy)ethane are preferable.

Examples of the trifunctional or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacrylate, 1,7-diacryloyloxy-2,2,6,6-tetraacryloyloxymethyl-4-oxyheptane, or the like.

The above monomers may be blended singly or in combination of multiple types.

The amount of the above-mentioned monomer that does not include an acidic group is preferably in the range of 10 parts by mass to 100 parts by mass, more preferably in the range of 20 parts by mass to 100 parts by mass, and still more preferably in the range of 50 parts by mass to 100 parts by mass, with respect to 100 parts by mass of the total amount of monomer components in the curable composition preparation kit of the present disclosure.

In addition, when the monomer component in the curable composition preparation kit of the present disclosure includes an acidic group-containing monomer described below, the amount of the monomer that does not include an acidic group is preferably from 10 parts by mass to 99 parts by mass, more preferably from 30 parts by mass to 97 parts by mass, and still more preferably from 50 parts by mass to 95 parts by mass, with respect to 100 parts by mass of the total amount of monomer components in the curable composition preparation kit of the present disclosure.

The monomer (A) and monomer (B) preferably contain a (meth)acrylic monomer (C) having a molecular weight of 100 to 5,000.

The molecular weight of the (meth)acrylic monomer (C) is more preferably from 120 to 3,000, still more preferably from 150 to 2,000, and particularly preferably from 200 to 1,000.

The content of the (meth)acrylic monomer (C) with respect to the total content of the monomer (A) and monomer (B) is preferably 50% by mass or more, more preferably 70% by mass or more, and still more preferably 90% by mass or more.

### (Acidic group-containing monomer)

In the curable composition preparation kit of the present disclosure, one of the monomer (A) contained in the first agent or the monomer (B) contained in the second agent preferably contains an acidic group-containing monomer.

Since at least one of the first agent or the second agent contains an acidic group-containing monomer, for example, when the curable composition preparation kit of the present disclosure is used for dental purposes, good tooth quality and high adhesion to a dental prosthetic material can be provided.

From the viewpoint of suppressing the reaction between an ascorbic acid-type compound or a phosphonite compound and an acidic group-containing monomer, it is preferable that the monomer (A) in the first agent contains an acidic group-containing monomer, and the second agent contains an ascorbic acid-type compound and a phosphonite compound, and it is more preferable that the monomer (A) in the first agent contains an acidic group-containing monomer, and the second agent does not include an acidic group-containing monomer and contains an ascorbic acid-type compound and a phosphonite compound.

Examples of the acidic group-containing monomer include a monomer that has at least one acidic group such as a phosphoric acid group, a pyrophosphoric acid group, a thiophosphoric acid group, a phosphonic acid group, a sulfonic acid group, or a carboxylic acid group, and that includes at least one polymerizable group such as an acryloyl group, a methacryloyl group, a vinyl group, or a styrene group.

The acidic group-containing monomer has an affinity with the adherend and has a demineralizing effect on the tooth substance.

Examples of a phosphate group-containing monomer include (meth)acryloyloxyalkyl dihydrogen phosphate such as 10-(meth)acryloyloxydecyl dihydrogen phosphate (MDP), bis[2-(meth)acryloyloxyethyl]hydrogen phosphate , bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9- (meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate and acid chlorides thereof, alkali metal salts thereof and ammonium salts thereof.

Examples of a pyrophosphate group-containing monomer include bis[2-(meth)acryloyloxyethyl] pyrophosphate, bis[4-(meth)acryloyloxybutyl] pyrophosphate, and acid chlorides thereof, alkali metal salts thereof, and ammonium salts thereof.

Examples of a thiophosphoric acid group-containing monomer include 2-(meth)acryloyloxyethyl dihydrogenthiophosphate, 3-(meth)acryloyloxypropyl dihydrogenthiophosphate, and acid chlorides thereof, alkali metal salts thereof, and ammonium salts thereof.

Examples of a phosphonic acid group-containing monomer include 2-(meth)acryloyloxyethyl phenylphosphonate, 5-(meth)acryloyloxypentyl-3-phosphonopropionate, and acid chlorides thereof, alkali metal salts thereof, and ammonium salts thereof.

Examples of a sulfonic acid group-containing monomer include 2-(meth)acrylamido-2-methylpropanesulfonic acid, styrenesulfonic acid, and 2-sulfoethyl (meth)acrylate.

Examples of a carboxylic acid group-containing monomer include a monomer containing one carboxy group in the molecule and a monomer containing multiple carboxy groups in the molecule.

Examples of the monomer containing one carboxy group in the molecule include (meth)acrylic acid, N-(meth)acryloylglycine, N-(meth)acryloyl aspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxybenzoic acid, ) acryloyloxyethyl hydrogen succinate, 2-(meth)acryloyloxyethyl hydrogen phthalate, 2-(meth)acryloyloxyethyl hydrogen malate, and acid halides thereof.

Examples of the monomer containing multiple carboxy groups in the molecule include 6-(meth)acryloyloxyhexane-1,1 -dicarboxylic acid, 9-(meth)acryloyloxynonane-1,1-dicarboxylic acid, 10-(meth)acryloyloxydecane-1,1-dicarboxylic acid. 11-(meth)acryloyloxyundecane-1,1-dicarboxylic acid, 12-(meth)acryloyloxydodecane-1,1-dicarboxylic acid, 13-(meth)acryloyloxy tridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, dihydroxyethyl methacrylate trimethylhexyldicarbamate, and acid anhydrides or acid halides thereof.

Among the above acidic group-containing monomers, from the point of high adhesive strength to an adherend, they are preferably 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2- methylpropanesulfonic acid, 11-(meth)acryloyloxyundecane-1,1-dicarboxylic acid, and dihydroxyethyl methacrylate trimethylhexyldicarbamate.

One type of the above acidic group-containing monomer may be used alone, or a plurality of types may be used in combination.

The amount of the acidic group-containing monomer is preferably from 1 parts by mass to 50 parts by mass, more preferably from 3 parts by mass to 40 parts by mass, and still more preferably from 5 parts by mass to 30 parts by mass, with respect to 100 parts by mass of the total amount of monomer components in the curable composition preparation kit of the present disclosure.

When the amount of the acidic group-containing monomer is 1 part by mass or more, it is easy to obtain high adhesiveness to various adherends.

Furthermore, when the amount of the acidic group-containing monomer is 50 parts by mass or less, it is easy to maintain a balance between polymerizability and adhesiveness. The total amount of monomer components means the total amount of the acidic group-containing monomer and the above-mentioned monomer that does not include an acidic group.

As for monomers in the present disclosure, for example, monomers described in known documents such as WO 2012/157566, WO 2015/015220, WO 2015/015221, and JP 2016-094482 can be used.

In the curable composition preparation kit of the present disclosure, each of the aforementioned phosphonite compound or the polymerization accelerator of the present disclosure, an oxidizing agent and a reducing agent are independently contained in at least one of the first agent or the second agent. Preferred embodiments of the aforementioned phosphonite compound or the polymerization accelerator, an oxidizing agent, and a reducing agent that are used in the curable composition preparation kit of the present disclosure are as described above.

In the curable composition preparation kit of the present disclosure, from the viewpoint of improving polymerizability, the oxidizing agent preferably contains an organic peroxide, and the reducing agent preferably contains an ascorbic acid-type compound and a transition metal compound.

In the curable composition preparation kit of the present disclosure, from the viewpoint of storage stability, the first agent preferably contains a transition metal compound and an organic peroxide, and the second agent preferably contains an ascorbic acid-type compound and a phosphonite compound.

The total content of the phosphonite compounds contained in the first agent and the second agent is preferably from 0.1% by mass to 5% by mass, and more preferably from 0.2% by mass to 3% by mass, and still more preferably from 0.3% by mass to 1% by mass, with respect to the total mass of the curable composition to be prepared.

The total content of the phosphonite compounds contained in the first agent and the second agent is preferably from 0.1% by mass to 1.5% by mass, and more preferably from 0.1% by mass to 1% by mass, and still more preferably from 0.2% by mass to 0.5% by mass, with respect to the total mass of the curable composition to be prepared.

The total content of the oxidizing agent contained in the first agent and the second agent is preferably from 0.1% by mass to 10% by mass, and more preferably from 0.3% by mass to 5% by mass, and still more preferably from 0.5% by mass to 3% by mass, with respect to the total mass of the curable composition to be prepared.

The total content of the reducing agent contained in the first agent and the second agent is preferably from 0.2% by mass to 5% by mass, and more preferably from 0.3% by mass to 2% by mass, and still more preferably from 0.5% by mass to 1.5% by mass, with respect to the total mass of the curable composition to be prepared.

The total content of ascorbic acid contained in the first agent and the second agent is preferably from 0.2% by mass to 5% by mass, more preferably from 0.3% by mass to 2% by mass, and still more preferably from 0.5% by mass to 1.5% by mass, with respect to the total mass of the curable composition to be prepared.

The total content of the transition metal compound contained in the first agent and the second agent is preferably from 0.0001% by mass to 0.1% by mass, and more preferably from 0.0002% by mass to 0.05% by mass, and still more preferably from 0.0003% by mass to 0.02% by mass, with respect to the total mass of the curable composition to be prepared.

### <Filler>

In the curable composition preparation kit of the present disclosure, at least one of the first agent or the second agent may contain a filler, and it is preferable that the first agent and the second agent contain a filler.

One type of filler may be blended alone, or a combination of multiple types may be blended. Examples of the filler include an inorganic filler, an organic filler, and a composite filler of an inorganic filler and an organic filler.

Examples of the inorganic filler include silica; a mineral based on silica, such as kaolin, clay, mica, or mica; a ceramic or a glass which contains Al₂O₃, B₂O₃, TiO₂, ZrO₂, BaO, La₂O₃, SrO, ZnO, CaO, P₂O₅, Li₂O, Na₂O or the like.

As the glasses, lanthanum glass, barium glass, strontium glass, soda glass, lithium borosilicate glass, zinc glass, fluoroaluminosilicate glass, borosilicate glass, or bioglass is suitably used.

Crystalline quartz, hydroxyapatite, alumina, titanium oxide, yttrium oxide, zirconia, calcium phosphate, barium sulfate, aluminum hydroxide, sodium fluoride, potassium fluoride, sodium monofluorophosphate, lithium fluoride, or ytterbium fluoride is also preferably used.

Specifically, fine particle silica having a primary particle diameter of 0.001 µm to 0.1 µm is preferably used in terms of adhesive strength and ease of handling. Examples of commercially available products include "Aerosil OX50", "Aerosil 50", "Aerosil 200", "Aerosil 380", "Aerosil R972", and "Aerosil 130" (all manufactured by Nippon Aerosil Co., Ltd.).

Examples of the organic filler include polymethyl methacrylate, polyethyl methacrylate, a polyfunctional methacrylate polymer, polyamide, polystyrene, polyvinyl chloride, chloroprene rubber, nitrile rubber, or styrene-butadiene rubber.

Examples of the composite filler of the inorganic filler and the organic filler include a filler in which an inorganic filler is dispersed in an organic filler, and an inorganic/organic composite filler in which an inorganic filler is coated with a various polymer.

In order to improve curability, mechanical strength, and handleability, the filler may be used after being previously surface-treated with a known surface treatment agent such as a silane coupling agent. Examples of the surface treatment agent include vinyltrimethoxysilane, vinyltriethoxysilane, vinyltrichlorosilane, vinyltri((β-methoxyethoxy)silane, γ-methacryloyloxypropyltrimethoxysilane, γ-glycidoxypropyltrimethoxysilane, γ-mercaptopropyltrimethoxysilane or γ-aminopropyltriethoxysilane.

The amount of the filler is preferably in the range of 10% by mass to 80% by mass, more preferably in the range of 30% by mass to 80% by mass, and still more preferably in the range of 50% by mass to 75% by mass, with respect to the total mass of the curable composition of the present disclosure.

### (Non-conductive filler)

In the curable composition preparation kit of the present disclosure, it is preferable that at least one of the first agent or the second agent contains a non-conductive filler.

A non-conductive filler means a filler with a resistance value of 1.00×10⁻⁴ Ωm or more.

The upper limit of the resistance value of the non-conductive filler is not particularly limited, and it may be, for example, 1.00×10²⁰ Ωm.

Examples of the material of the non-conductive filler include an organic material such as polyethylene, polystyrene, a phenol resin, an epoxy resin, an acrylic resin, or a benzoguanamine resin; silica (dimethylsilylated silica, or the like); a silicate (borosilicate glass (barium borosilicate glass, or the like)); an aluminosilicate glass (boroaluminosilicate glass, strontium boroaluminosilicate glass, fluoroaluminosilicate glass, barium aluminosilicate glass, or the like), a ceramic, an inorganic material such as boron nitride or barium nitride or the like.

Among the above materials, the materials of the non-conductive filler are preferably silica and silicate, and more preferably dimethylsilylated silica and barium aluminosilicate.

The first agent contains a non-conductive filler, and the content of the non-conductive filler with respect to the total mass of the first agent is preferably 10% by mass or more, more preferably 20% by mass or more, and still more preferably 30% by mass or more.

When the second agent contains a non-conductive filler, the second agent contains the non-conductive filler, and the content of the non-conductive filler with respect to the total mass of the second agent is preferably 10% by mass or more, more preferably 20% by mass or more, and still more preferably 30% by mass or more.

In the curable composition preparation kit of the present disclosure, when the non-conductive filler is contained in the first agent, the content of the non-conductive filler with respect to the total mass of the first agent is preferably 10% by mass or more or when the non-conductive filler is contained in the second agent, the content of the non-conductive filler with respect to the total mass of the second agent is preferably 10% by mass or more.

### <Additive>

The curable composition of the present disclosure may contain an additive such as a photopolymerization initiator, a stabilizer (polymerization inhibitor), a colorant, a fluorescent agent, an ultraviolet absorber or the like.

As the photopolymerization initiator, known photopolymerization initiators can be used, examples thereof include camphorquinone (CQ), ethyl dimethylaminobenzoate (EDB), 2-butoxyethyl 4-(dimethylamino)benzoate (BEDB) or the like.

In addition, an antibacterial substance such as cetylpyridinium chloride, benzalkonium chloride, (meth)acryloyloxydodecylpyridinium bromide, (meth)acryloyloxyhexadecylpyridinium chloride, (meth)acryloyloxydecyl ammonium chloride, or triclosan may be blended.

A known dye or pigment may be blended into the curable composition of the present disclosure.

The curable composition preparation kit of the present disclosure is preferably used for a dental material.

The dental material is not particularly limited, and examples thereof include a dental adhesive, dental filling material, dental sealant (tooth fissure sealant), abutment building material, denture base resin, denture base lining material, dental crown prosthesis resin (hard resin for dental crown), dental room temperature polymerization resin, or the like.

The curable composition preparation kit of the present disclosure is particularly preferably used as a dental adhesive.

Examples of the dental adhesive include dental adhesive resin cement, orthodontic adhesive, adhesives for fixing loose teeth, cavity coating adhesive, and dental bonding material, or the like, and dental adhesive resin cement is preferable.

Examples of the dental filling material include a dental composite resin (including dental self-adhesive composite resin), root canal filling material, temporary sealant, backing material, or the like.

The curable composition preparation kit of the present disclosure may be used for dental treatment.

For example, the dental treatment method of the present disclosure may include a step of mixing the first agent and the second agent in the curable composition preparation kit of the present disclosure to obtain a curable composition, and a step of polymerizing the curable composition in an oral cavity to obtain a cured product. In the method, the step of mixing the first agent and the second agent to obtain a curable composition may be performed inside the oral cavity or outside the oral cavity. When performing outside the oral cavity, the obtained curable composition may be applied to the oral cavity and polymerized inside the oral cavity to obtain a cured product. As described above, the method including the step of polymerizing in the oral cavity to obtain a cured product is suitable, for example, when the curable composition preparation kit is used for dental adhesive resin cement, composite resin for filling and restoration, or the like.

The dental treatment method of the present disclosure may include a step of polymerizing the curable composition of the present disclosure outside the oral cavity to obtain a cured product, and a step of applying the cured product inside the oral cavity. The step of polymerizing outside the oral cavity to obtain a cured product may be a step of polymerizing the curable composition in a cast mold to obtain a cured product. The cured product obtained outside the oral cavity may be processed as necessary, and the cured product after processing may be applied inside the oral cavity. The method of obtaining a cured product by polymerizing outside the oral cavity is suitable, for example, when the cured product is used for a CAD/CAM resin block, temporary crown, artificial tooth, or the like.

### <Curable Composition>

The curable composition of the present disclosure contains the aforementioned phosphonite compound or the polymerization accelerator of the present disclosure, an oxidizing agent, a reducing agent, and a monomer. When the curable composition of the present disclosure contains the above components, polymerizability can be favorably improved.

Specific examples, preferred embodiments, or the like of the monomer in the curable composition are the same as those for the above-mentioned monomer.

It is preferable that the curable composition of the present disclosure further contains a non-conductive filler.

Specific examples, preferred embodiments, or the like of the non-conductive filler in the curable composition are the same as those for the above-mentioned non-conductive filler.

### <Cured Product>

The cured product of the present disclosure is a cured product of the curable composition of the present disclosure, or a cured product obtained using the curable composition preparation kit of the present disclosure.

The cured product of the present disclosure can be suitably used as a dental material. That is, the dental material of the present disclosure preferably includes the cured product of the present disclosure.

### EXAMPLES

Examples of the present disclosure will be shown below. This disclosure is not limited by the following examples.

Each component used in the examples is shown below.

### <Polymerization accelerator>

PPP: Tetrakis(2,4-di-tert-butylphenyl)4,4'-biphenylene-di-phosphonite
TPP: Triphenyl phosphite
PS: Di-n-propyl sulfite
NaCl: Sodium chloride

### <Transition metal compound>

Cu(OAc)₂ monohydrate: copper acetate monohydrate

### <Filler>

GM27884: GM27884 manufactured by Schott, 1.5 µm, silane coupling agent processing amount 2.3%
R812: AEROSIL(R)R812 manufactured by Evonik Industries AG

### <Ascorbic Acid-type Compound>

isoC4AAA: 3,4-dihydroxy-5-(2-isopropyl-1,3-dioxolan-4-yl)furan-2(5H)-one (see the following chemical formula)

### <Monomer that does not include acidic group>

2-HBMA: 2-hydroxybutyl methacrylate
TEGDMA: Triethylene glycol dimethacrylate
UDMA: 2,2,4-trimethylhexamethylenebis(2-carbamoyloxyethyl)dimethacrylate

### <Acidic group-containing monomer>

MDP: 10-methacryloyloxydecyl dihydrogen phosphate

### <Organic peroxide>

TAH: Tert-amyl hydroperoxide

### <Photopolymerization initiator>

BEDB: 2-butoxyethyl 4-(dimethylamino)benzoate
CQ: Camphorquinone

### <Polymerization inhibitor>

BHT: 2,6-di-tert-butyl-p-cresol

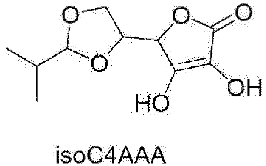

### <Synthesis Example 1 (isoC4AAA: Synthesis of 3,4-dihydroxy-5-(2-isopropyl-1,3-dioxolan-4-yl)furan-2(5H)-one)>

Ascorbic acid (50.0 g, 284 mmol) and dimethylacetamide (105 mL) as a solvent were charged into a device connected to a 300 mL eggplant-bottomed flask and a Dimroth condenser, and the temperature was raised to 60°C and the solution was stirred uniformly.

Then, isobutanal (20.2 g, 284 mmol) was added to the solution and the solution was stirred uniformly. Furthermore, p-toluenesulfonic acid monohydrate (5.7 g, 28 mmol), which is an acid catalyst, was added to the solution, and the solution was stirred uniformly. After 20 hours, the reaction was stopped and the mixture was cooled to room temperature.

Thereafter, the obtained reaction product was extracted. Specifically, an equal amount of distilled water was added to the reaction solution, then an equal amount of diethyl ether was added, and the mixture was extracted and washed using a separatory funnel. After repeating the above extraction three times, an equal amount of distilled water was added to the obtained organic layer and washed with a separatory funnel. After repeating the above washing three times, the resulting organic layer was dried by adding sodium sulfate, and then concentrated under reduced pressure. The obtained crude product was washed with a hexane/ethyl acetate solvent, and separated and purified by filtration.

Through the above steps, 3,4-dihydroxy-5-(2-isopropyl-1,3-dioxolan-4-yl)furan-2(5H)-one (16 g, 70 mmol, yield 24.6%) was obtained. The attribution by ¹H-NMR(CD₃OD) of isoC4AAA is shown below.
¹H-NMR:50.39(td,6H,J=4.5Hz,2.2Hz),1.19-1.30(m,1H),3.39-3.79(m,3H),4.10-4.19(m,2H)

As shown in Table 1, the first agent and the second agent of Example 1 and Comparative Examples 1 to 3 were prepared using each component. In Table 1, the numbers in each component column mean the amount (parts by mass) of each component in the first agent or the second agent.

### <Polymerizability evaluation>

100 mg of each of the first agent and the second agent were weighed and kneaded for 20 seconds. Approximately 100 mg of the kneaded composition was immediately filled into an aluminum pan, and measurement was performed using DSC Sirius manufactured by NETZSCH. The peak was analyzed, and the peak top (time (minutes) at which the maximum value of the peak was obtained) and Height (mW/mg) were calculated.

Peak top (minutes) is an index of polymerization rate, and Height (mW/mg) is an index of degree of polymerization. These evaluation criteria are as follows.

### -Evaluation criteria for peak top (minutes)-

A 2.0 or less
B More than 2.0 and less than 2.4
C 2.4 or more and 3.0 or less
D More than 3.0

### -Evaluation criteria for Height (mW/mg)-

A 1.6 or more
B More than 1.3 and less than 1.6
C 0.6 or more and 1.3 or less
D less than 0.6

**[Table 1]**

| | | Example | | Comparative Example | | | | | |
|---|---|---|---|---|---|---|---|---|---|
| | | 1 | | 1 | | 2 | | 3 | |
| | | First agent | Second agent | First agent | Second agent | First agent | Second agent | First agent | Second agent |
| Polymerization accelerator | PPP | | 0.500 | | | | | | |
| | TPP | | | | 0.500 | | | | |
| | PS | | | | | | 0.500 | | |
| | NaCl | | 0.015 | | 0.015 | | 0.015 | | 0.015 |
| Transition metal compound | Cu(OAc)₂ monohydrate | 0.001 | | 0.001 | | 0.001 | | 0.001 | |
| Filler | GM27884 | 57.237 | 65.067 | 57.237 | 65.067 | 57.237 | 65.067 | 57.237 | 65.400 |
| | R812 | 1.321 | 1.481 | 1.321 | 1.481 | 1.321 | 1.481 | 1.321 | 1.489 |
| Ascorbic acid-type compound | C4iso AAA | | 1.500 | | 1.500 | | 1.500 | | 1.500 |
| Monomer that does not include acidic group | 2-HBMA | 1.761 | 1.974 | 1.761 | 1.974 | 1.761 | 1.974 | 1.761 | 1.984 |
| | TEGDMA | 7.925 | 8.885 | 7.925 | 8.885 | 7.925 | 8.885 | 7.925 | 8.931 |
| | UDMA | 17.710 | 20.328 | 17.710 | 20.328 | 17.710 | 20.328 | 17.710 | 20.431 |
| Acidic group-containing monomer | MDP | 10.000 | | 10.000 | | 10.000 | | 10.000 | |
| Organic peroxide | TAH | 4.000 | | 4.000 | | 4.000 | | 4.000 | |
| Photopolymerization initiator | BEDB | | 0.100 | | 0.100 | | 0.100 | | 0.100 |
| | CQ | | 0.100 | | 0.100 | | 0.100 | | 0.100 |
| Polymerization inhibitor | BHT | 0.045 | 0.050 | 0.045 | 0.050 | 0.045 | 0.050 | 0.045 | 0.050 |
| Total | | 100.000 | 100.000 | 100.000 | 100.000 | 100.000 | 100.000 | 100.000 | 100.000 |
| Polymerizability | Polymerization rate | A | | C | | D | | C | |
| | Degree of polymerization | A | | C | | D | | C | |

As shown in Table 1, in Example 1, which uses a phosphonite compound, the evaluation of polymerizability was better than in Comparative Examples 1 to 3.

The entire contents of the disclosures by Japanese Patent Application No. 2022-050803 filed on March 25, 2022, are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

## Claims

1. A polymerization accelerator, comprising a phosphonite compound.

2. The polymerization accelerator according to claim 1, wherein the phosphonite compound includes a structure represented by the following general formula (X): 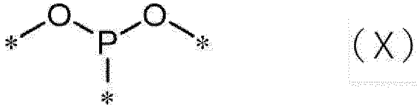 wherein, in the general formula (X), * is a bonding position with a carbon atom.

3. The polymerization accelerator according to claim 1 or 2, wherein the phosphonite compound contains a compound represented by the following general formula (Y): 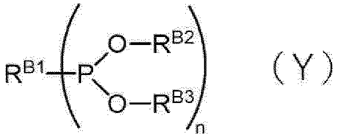 wherein, in the general formula (Y), R^{B1} represents an n-valent hydrocarbon group, each of R^{B2} and R^{B3} independently represents a monovalent hydrocarbon group, and n is an integer 1 or 2.

4. A polymerization initiator, comprising the phosphonite compound according to any one of claims 1 to 3, an oxidizing agent and a reducing agent.

5. A curable composition preparation kit, comprising:
a first agent containing a monomer (1), and
a second agent containing a monomer (2),
wherein each of the phosphonite compound according to any one of claims 1 to 3, an oxidizing agent and a reducing agent are independently contained in at least one of the first agent or the second agent.

6. The curable composition preparation kit according to claim 5, wherein:
the oxidizing agent contains an organic peroxide, and
the reducing agent contains an ascorbic acid-type compound and a transition metal compound.

7. The curable composition preparation kit according to claim 6, wherein:
the first agent contains the transition metal compound and the organic peroxide, and
the second agent contains the ascorbic acid-type compound and the phosphonite compound.

8. The curable composition preparation kit according to any one of claims 5 to 7, wherein one of the monomer (1) contained in the first agent or the monomer (2) contained in the second agent contains an acidic group-containing monomer.

9. The curable composition preparation kit according to claim 6 or 7, wherein:
the monomer (1) contained in the first agent contains an acidic group-containing monomer, and
the second agent contains the ascorbic acid-type compound and the phosphonite compound.

10. The curable composition preparation kit according to any one of claims 6 to 9, wherein the second agent contains the ascorbic acid-type compound and the phosphonite compound.

11. The curable composition preparation kit according to any one of claims 5 to 10, wherein the first agent and the second agent contain a filler.

12. The curable composition preparation kit according to any one of claims 5 to 11, wherein a total content of the phosphonite compound contained in the first agent and the second agent is from 0.1% by mass to 1.5% by mass with respect to a total amount of a curable composition to be prepared.

13. A curable composition, comprising the phosphonite compound according to any one of claims 1 to 3, an oxidizing agent, a reducing agent and a monomer.

14. A cured product of the curable composition according to claim 13.

15. A dental material, comprising the cured product according to claim 14.
